# EUROPEAN PATENT APPLICATION

(11) **EP 4 635 458 A1**
(43) Date of publication of application: **22.10.2025**
(21) Application number: 24173341.9
(22) Date of filing: 30.04.2024
(51) Int. Cl.: A61F 7/00, H05B 3/06

(54) **COMBINED TYPE HAND WARMER**

(30) Priority: 15.04.2024 CN 202420764166 U
(71) Applicant: Shenzhen Yise Technology Co., Ltd, Shenzhen (CN)
(72) Inventor: Deng, Lixin, Shenzhen (CN)
(74) Representative: Cleanthous, Marinos

(57) **Abstract**

A combined type hand warmer is provided, including a first heating unit, a second heating unit, a connection portion, and a locking portion, wherein the connection portion includes an insertion block located on the first heating unit and a connection block located on the second heating unit; the connection block is provided with an insertion slot matched with the insertion block; the locking portion includes a movement hole located on an inner surface of the first heating unit, a lock hole located on an inner surface of the second heating unit, and an elastic sheet mounted in the first heating unit; and the elastic sheet is provided with a locking piece located in the movement hole and matched with the lock hole.

## Description

### TECHNICAL FIELD

The present disclosure belongs to the technical field of hand warmers, and in particular, to a combined type hand warmer.

### BACKGROUND

A hand warmer is an electronic product that can serve as a heat source, which mainly generates heat for warming through a heating sheet inside a shell. Most of the traditional hand warmers are provided with a single unit, which cannot be used to warm both hands/multiple body parts, making it difficult to meet the needs of users. However, if a user carries multiple single-unit hand warmers for warming when going out, these hand warmers would occupy a large space and have poor portability.

For this purpose, patent No. 2023220780509 provides a combined type hand warmer power bank, which includes a first branch body and a second branch body. The first branch body and the second branch body are provided with a matched fastener body and fastener body slot for preliminary limitation; two ends of the fastener body are also provided with clamping buckles; two ends of the fastener body slot are provided with clamping slots; and the clamping buckles and the clamping slots are clamped to each other to detachably connect the first branch body to the second branch body.

This solution can solve the problems of the traditional hand warmer, but in actual use, it has been found that pulling the first branch body and the second branch body can easily loosen the clamping buckles, so that the structural stability is poor; and the exposed clamping buckles are prone to collision and damage, resulting in a short service life.

### SUMMARY

### (I) Technical problems to be solved

The present disclosure provides a combined type hand warmer, which can improve the structural stability and prolong the service life.

### (II) Technical solutions

In order to achieve the above objective, the present disclosure provides the following technical solutions:
A combined type hand warmer includes: a first heating unit, a second heating unit, a connection portion, and a locking portion, wherein the connection portion includes an insertion block and a connection block; the insertion block is arranged on an edge of the first heating unit and extends laterally; the connection block is arranged on an edge of the second heating unit and is provided with an insertion slot with a lateral opening; the insertion block can be separated from or inserted into the insertion slot; and the locking portion includes a movement hole, an elastic sheet, and a lock hole; the movement hole is arranged on an inner surface of the first heating unit; the lock hole is arranged on an inner surface of the second heating unit; the elastic sheet is mounted inside the first heating unit; the elastic sheet is provided with a locking piece; and the locking piece is located at the movement hole and can be separated from or clamped into the lock hole.

Preferably, the insertion block on the first heating element protrudes out of the inner surface of the first heating element; the inner surface of the second heating element is provided with a first guide port that is inwards sunken; and the insertion block on the first heating element is able to pass through the first guide port and be inserted into the insertion slot on the second hand warmer unit.

Preferably, a first hidden slot is arranged on the first heating unit; and when the connection block on the second heating unit is placed in the first hidden slot, the insertion block on the first heating unit is inserted into the insertion slot on the second heating unit.

Preferably, the combined type hand warmer includes two connection portions; the insertion blocks of the two connection portions are respectively arranged on an edge of the first heating unit and an edge of the second heating unit; the connection blocks of the two connection portions are respectively arranged on the first heating unit and the second heating unit; and when the insertion block on the first heating unit is inserted into the insertion slot on the second heating unit, the insertion block on the second heating unit is synchronously inserted into the insertion slot on the first heating unit.

Preferably, the insertion block on the first heating unit protrudes out of the inner surface of the first heating unit; the inner surface of the second heating unit is provided with a first guide port that is inwards sunken; the insertion block on the first heating unit can pass through the first guide port and be inserted into the insertion slot on the second heating unit; the insertion block on the second heating unit protrudes out of the inner surface of the second heating unit; the inner surface of the first heating unit is provided with a second guide port that is inwards sunken; and the insertion block on the second heating unit can pass through the second guide port and be inserted into the insertion slot on the first hand warmer unit.

Preferably, a first hidden slot is arranged on the first heating unit; when the connection block on the second heating unit is placed in the first hidden slot, the insertion block on the first heating unit is inserted into the insertion slot on the second heating unit; a second hidden slot is arranged on the second heating unit; and when the connection block on the first heating unit is placed in the second hidden slot, the insertion block on the second heating unit is inserted into the insertion slot on the first heating unit.

Preferably, the combined type hand warmer includes two locking portions; the elastic sheets of the two locking portions are respectively mounted inside the first heating unit and the second heating unit, and the two elastic sheets are staggered from each other; and the movement holes and lock holes of the two locking portions are distributed on the inner surface of the first heating unit and the inner surface of the second heating unit to respectively adapt to the locking pieces of the two elastic sheets.

Preferably, the combined type hand warmer includes four locking portions to respectively correspond to top and bottom sides of the first heating unit and top and bottom sides of the second heating unit.

Preferably, the locking pieces are hemispherical.

Preferably, the first heating unit includes a shell, a shell cover, a battery, and a heating element; the shell cover is mounted on the shell and is enclosed to form an accommodating cavity; the battery and the heating element are both mounted in the accommodating cavity; the battery is electrically connected to the heating element; and the second heating unit has the same structure as that of the first heating unit.

### (III) Beneficial effects

According to the combined type hand warmer provided by the present disclosure, by the design of the connection portion and the locking portion which are matched with each other to connect and lock the first heating unit with the second heating unit, so as to achieve assembling and removal between the first heating unit and the second heating unit, which improves the universality. In the assembling and removal processes, the inner surface of the first heating unit and the inner surface of the second heating unit abut against each other, and the locking piece on the first heating unit only needs to bear a pressure applied by the second heating unit to adaptively deform, so that the pressure on the elastic sheet is low, and the elastic sheet deforms little. This can reduce the possibility of looseness and improve the structural stability. After the first heating unit is separated from the second heating unit, the elastic sheet is reset and drives the locking piece to protrude out of the movement hole to the outside. However, since the elastic sheet has the deformation ability, the exposed locking piece may quickly contract into the movement hole when it is in contact with an object, to prevent damage caused by rigid collision between the elastic sheet and the object, and the service life is prolonged.

### BRIEF DESCRIPTION OF THE DRAWINGS

The accompanying drawings are intended to provide a further understanding of the present disclosure and constitute a part of this specification. The accompanying drawings and the embodiments of the present disclosure are used together for explaining the present disclosure rather than constituting a limitation on the present disclosure. In the drawings:
FIG. 1 shows a schematic diagram of a hand warmer after combination;
FIG. 2 shows a top view of FIG. 1;
FIG. 3 shows a sectional view A-A of FIG. 2;
FIG. 4 shows an enlarged view of the part A in FIG. 3;
FIG. 5 shows a sectional view B-B of FIG. 2;
FIG. 6 shows an enlarged view of the part B in FIG. 5;
FIG. 7 shows a schematic diagram I of a hand warmer after separation;
FIG. 8 shows a schematic diagram II of a hand warmer after separation;
FIG. 9 shows a schematic exploded diagram of FIG. 7;
FIG. 10 shows an enlarged view of the part C in FIG. 9;
FIG. 11 shows a schematic exploded diagram of a first heating unit; and
FIG. 12 shows an enlarged view of the part D in FIG. 11.

In the drawings: 1: first heating unit; 10: shell; 100: accommodating cavity; 101: first hidden slot; 102: second guide port; 11: shell cover; 12: battery; 13: heating element; 131: circuit board; 132: circuit board; 133: control button; 134: charging end; 135; display lamp; 2: second heating unit; 201: first guide port; 202: second hidden slot; 3: connection portion; 31: insertion block; 32: connection block; 320: insertion slot; 4: locking portion; 41: movement hole; 42: lock hole; 43: elastic sheet; and 430: locking piece.

### DETAILED DESCRIPTION OF THE EMBODIMENTS

The technical solutions in embodiments of the present disclosure are clearly and completely described in the following with reference to the accompanying drawings in the embodiments of the present disclosure. Apparently, the described embodiments are merely some rather than all of the embodiments of the present disclosure. All other embodiments obtained by a person of ordinary skill in the art based on the embodiments of the present disclosure without making creative efforts shall fall within the protection scope of the present disclosure. It can be understood that the drawings are only for reference and illustration purposes and are not intended to limit the present disclosure. The connection relationship shown in the accompanying drawings is only for the sake of clear description and does not limit a connection method.

It should be noted that when an assembly is considered to be "connected" to another assembly, the assembly can be directly connected to the another assembly or there may be an intermediate assembly. Unless otherwise defined, all technical and scientific terms used herein have the same meaning as those commonly understood by a person skilled in the art to which the present disclosure belongs. It should be also noted that unless otherwise specified and limited, the terms "mount", "connect", and "connection" should be broadly understood. For example, it can be a fixed connection, detachable connection, integrated connection, mechanical connection, electrical connection, or internal communication between two elements. A person of ordinary skill in the art can understand the specific meanings of the above terms in the present disclosure according to specific situations. Terms used in the specification of the present disclosure herein are merely intended to describe objectives of the specific embodiments, but are not intended to limit the present disclosure.

It should be also noted that in the descriptions of the present disclosure, orientations or positional relationships indicated by the terms "center", "upper", "lower", "left", "right", "vertical", "horizontal", "inner", "outer", and the like are orientations or positional relationships as shown in the drawings, and are only for the purpose of facilitating and simplifying the descriptions of the present disclosure instead of indicating or implying that devices or elements indicated must have particular orientations, and be constructed and operated in the particular orientations, so that these terms are not construed as limiting the present disclosure. In addition, the terms "first" and "second" are only for the purpose of description, and may not be understood as indicating or implying the relative importance.

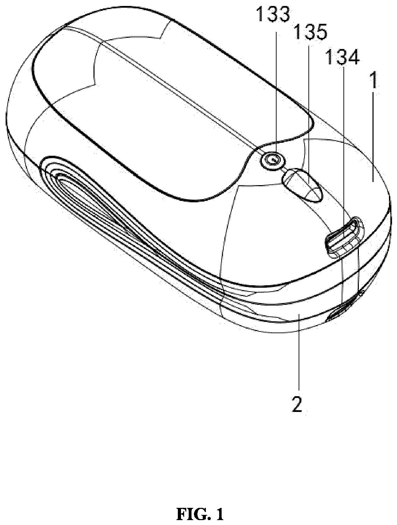

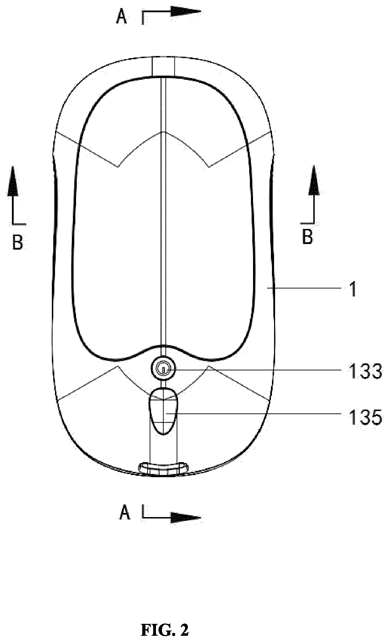

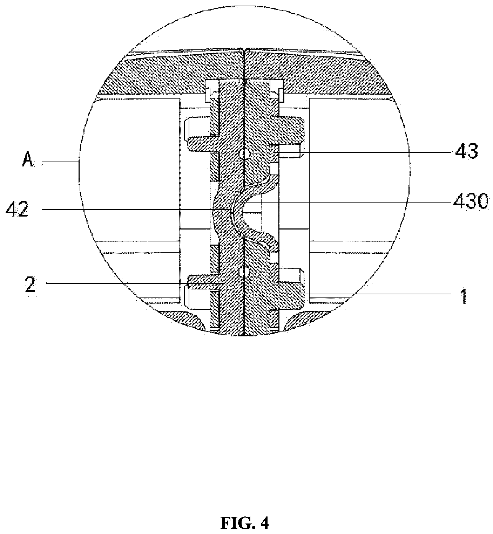

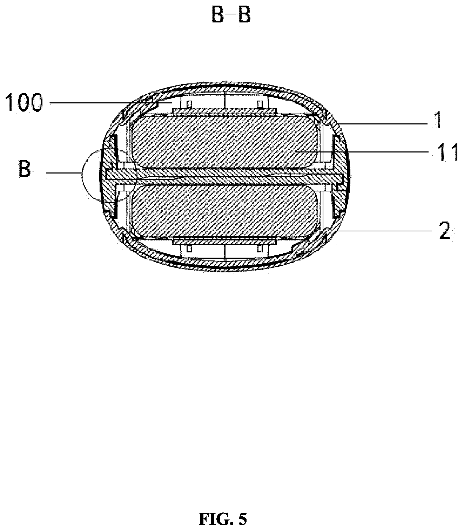

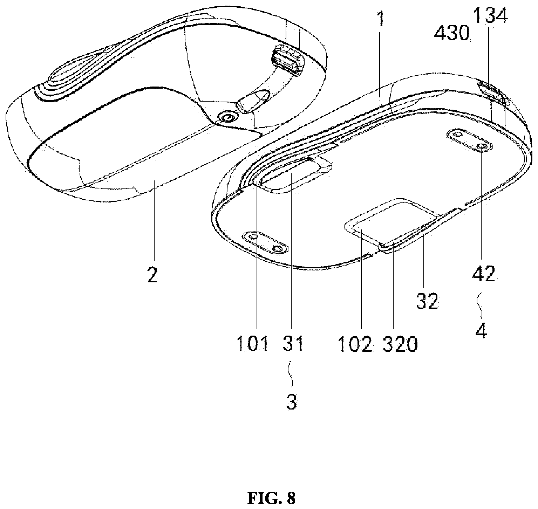

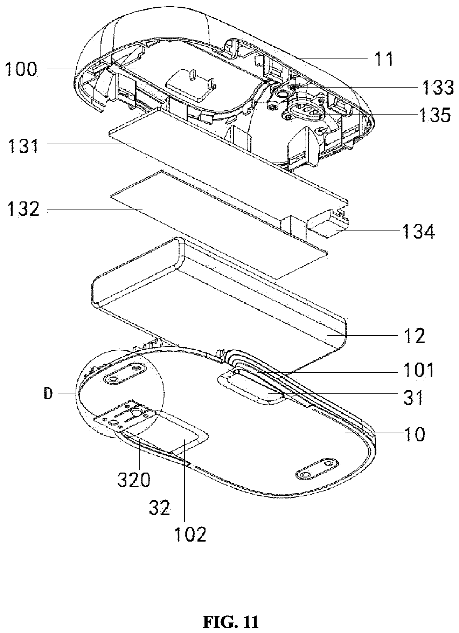

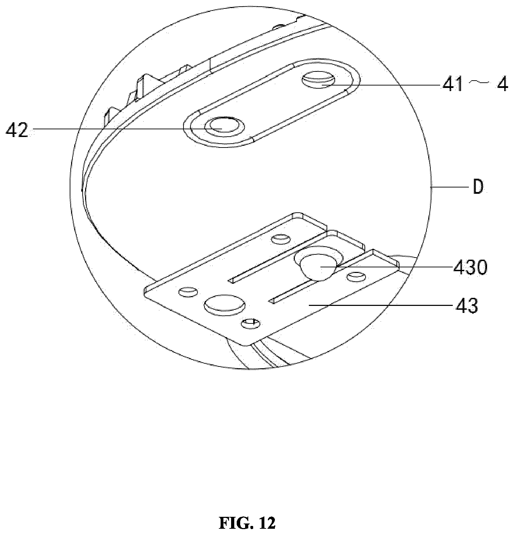

Referring to FIG. 1 to FIG. 8 and FIG. 11 to FIG. 12, a combined type hand warmer includes: a first heating unit 1, a second heating unit 2, a connection portion 3, and a locking portion 4, wherein the connection portion 3 includes an insertion block 31 and a connection block 32; the insertion block 31 is arranged on an edge of the first heating unit 1 and extends laterally; the connection block 32 is arranged on an edge of the second heating unit 2 and is provided with an insertion slot 320 with a lateral opening; the insertion block 31 can be separated from or inserted into the insertion slot 320; and the locking portion 4 includes a movement hole 41, a lock hole 42, and an elastic sheet 43; the movement hole 41 is arranged on an inner surface of the first heating unit 1; the lock hole 42 is arranged on an inner surface of the second heating unit 2; the elastic sheet 43 is mounted inside the first heating unit 1; the elastic sheet 43 is provided with a locking piece 420; and the locking piece 430 is located at the movement hole 41 and can be separated from or clamped into the lock hole 42.

Specifically, under normal circumstances, the insertion block 31 is inserted into the insertion slot 320 of the connection block 32 to limit relative rotation and longitudinal/vertical movement between the first heating unit 1 and the second heating unit 2. Meanwhile, under the action of the elastic sheet 43, the locking piece 430 is clamped into the lock hole 42 to limit the lateral/vertical movement of the first heating unit 1 and the second heating unit 2, thereby combining the first heating unit 1 and the second heating unit 2 to form the hand warmer for easy carrying.

When the first heating unit 1 or the second heating unit 2 is pulled laterally, the elastic sheet 43 may deform and be compressed under the pushing of the inner surface of the second heating unit 2, so that the locking piece 430 is gradually separated from the lock hole 42 and enters the movement hole 41. Meanwhile, the insertion block 31 may also be separated from the insertion slot 320 to relieve the connection restriction between the first heating unit 1 and the second heating unit 2, so that the first heating unit 1 and the second heating unit 2 can be used separately.

If the first heating unit 1 needs to be combined with the second heating unit 2 again, the inner surface of the first heating unit 1 first abuts against the inner surface of the second heating unit 2, and the locking piece 430 is then pushed into the movement hole 41 by the inner surface of the second heating unit 2. Next, the first heating unit 1 or the second heating unit 2 is moved laterally until the insertion block 31 is inserted into the insertion slot 320 on the connection block 32, and the locking piece 430 is reset to protrude out of the movement hole 41 and be clamped into the lock hole 42, so that the first heating unit 1 and the second heating unit 2 are connected and fixed.

In summary, by the design of the connection portion 3 and the locking portion 4 which are matched with each other to connect and lock the first heating unit 1 with the second heating unit 2, so as to achieve assembling and removal between the first heating unit and the second heating unit, which improves the universality. In the assembling and removal processes, the inner surface of the first heating unit 1 and the inner surface of the second heating unit 2 abut against each other, and the elastic sheet 43 inside the first heating unit 1 only needs to bear a pressure applied by the second heating unit 2 to the locking piece 430 to adaptively deform, so that the pressure on the elastic sheet 43 is low, and the elastic sheet deforms little. This can reduce the possibility of looseness and improve the structural stability. After the first heating unit 1 is separated from the second heating unit 2, the elastic sheet 43 is reset and drives the locking piece 430 to protrude out of the movement hole 41 to the outside. However, since the elastic sheet 43 has the deformation ability, the exposed locking piece 430 may quickly contract into the movement hole 41 when it is in contact with an object, to prevent damage caused by rigid collision between the elastic sheet 43 and the object, and the service life is prolonged.

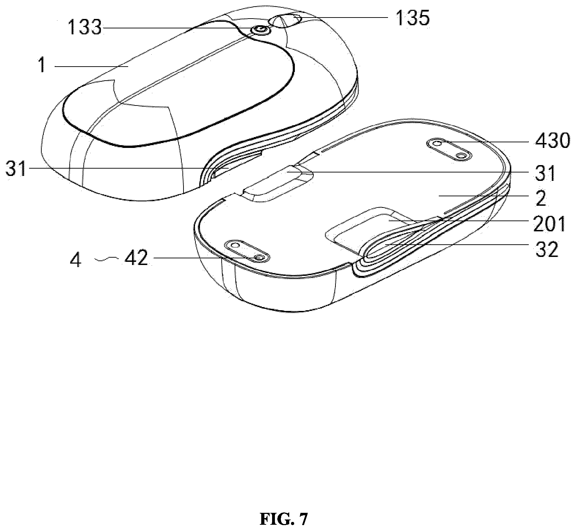

Referring to FIG. 7 to FIG. 8, the insertion block 31 on the first heating element 1 protrudes out of the inner surface of the first heating element 1; the inner surface of the second heating element 2 is provided with a first guide port 201 that is inwards sunken; and the insertion block 31 on the first heating element 1 can pass through the first guide port 201 and be inserted into the insertion slot 320 on the second heating unit.

Specifically, to assemble the hand warmer, the inner surface of the first heating unit 1 abuts against the inner surface of the second heating unit 2, and a protruding portion of the insertion block 31 is placed in the first guide port 201. The first guide port 201 can limit and guide the insertion block 31, so that the insertion block 31 can be accurately and quickly inserted into the insertion slot 320 of the connection block 32.

To disassemble the hand warmer, the first heating unit 1 or the second heating unit 2 is pushed laterally until there is obvious resistance after the insertion block 31 is completely separated from the insertion slot 320 and enters the first guide port 201, so as to prompt a user to remove the second heating unit 2 from the first heating unit 1 longitudinally.

Therefore, the cooperative use of the insertion block 31 on the first heating unit 1 and the first guide port 201 can ensure the assembling accuracy between the first heating unit 1 or the second heating unit 2, improve the convenience of use, and provide a user with a feedback to indicate a current position of the first heating unit 1 or the second heating unit 2, making it convenient for the user to disassemble the hand warmer and improving the operating handfeel.

Referring to FIG. 7 to FIG. 8, a first hidden slot 101 is arranged on the first heating unit 1; when the connection block 32 on the second heating unit 2 is placed in the first hidden slot 101, the insertion block 31 on the first heating unit 1 is inserted into the insertion slot 320 on the second heating unit 2; and when the connection block 32 on the second heating unit 2 is away from the first hidden slot 101, the insertion block 31 on the first heating unit 1 is separated from the insertion slot 320 on the second heating unit 2.

Since the first hidden slot 101 can sleeve and limit the connection block 32 on the second heating unit 2, on the one hand, this can improve the stability of connection between the first heating unit 1 and the second heating unit 2 after assembling; and on the other hand, this can also avoid a protruding portion on an edge of the assembled hand warmer, thus ensuring the overall aesthetics and making it convenient for a user to hold and carry the hand warmer.

Referring to FIG. 1 to FIG. 8, the combined type hand warmer includes two connection portions 3; the insertion blocks 31 of the two connection portions 3 are respectively arranged on an edge of the first heating unit 1 and an edge of the second heating unit 2; the connection blocks 32 of the two connection portions 3 are respectively arranged on the first heating unit 1 and the second heating unit 2; and when the insertion block 31 on the first heating unit 1 is inserted into the insertion slot 320 on the second heating unit 2, the insertion block 31 on the second heating unit 2 is synchronously inserted into the insertion slot 320 on the first heating unit 1.

Specifically, to assemble the hand warmer, the inner surface of the first heating unit 1 abuts against the inner surface of the second heating unit 2, so that the insertion block 31 and insertion slot 320 on the first heating unit 1 are aligned with the insertion slot 320 and insertion block 31 on the second heating unit 2, respectively. Next, the first heating unit 1 or the second heating unit 2 is moved laterally, so that the corresponding insertion blocks 31 and insertion slots 320 are inserted into each other. At this time, the locking piece 430 is inserted into the lock hole 42, so that the first heating unit 1 and the second heating unit 2 are fixed to each other. Therefore, the design of the two connection portions 3 can simultaneously limit the two opposite edges of the first heating unit 1 and the second heating unit 2, so as to further improve the overall structural stability.

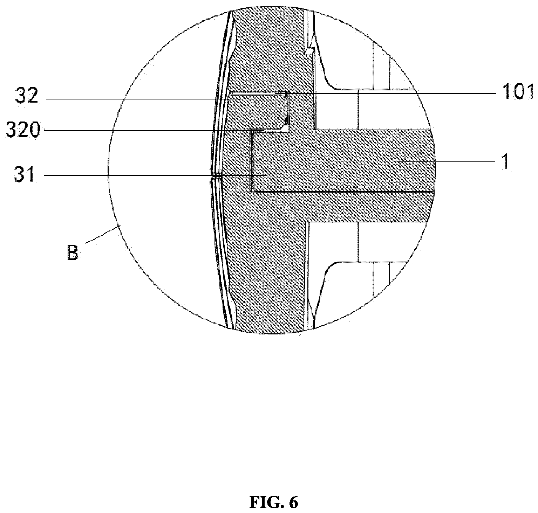

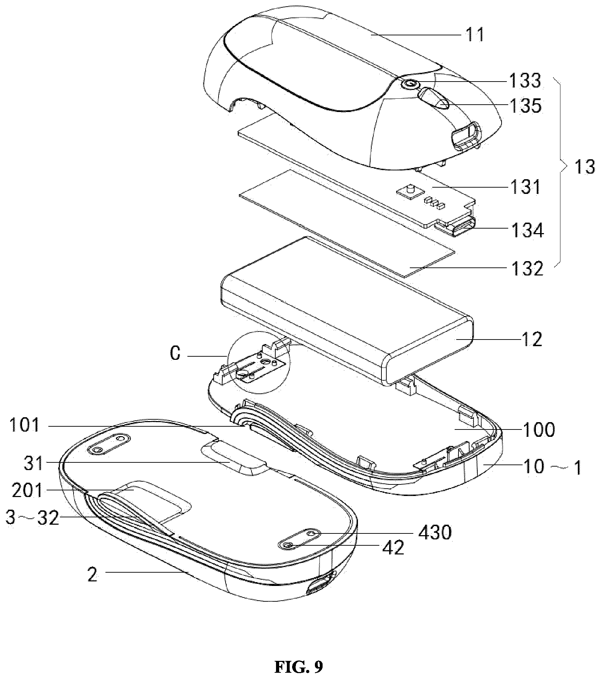

Referring to FIG. 6 to FIG. 9, the insertion block 31 on the first heating unit 1 protrudes out of the inner surface of the first heating unit 1; the inner surface of the second heating unit 2 is provided with a first guide port 201 that is inwards sunken; the insertion block 31 on the first heating unit 1 can pass through the first guide port 201 and be inserted into the insertion slot 320 on the second heating unit; the insertion block 31 on the second heating unit 2 protrudes out of the inner surface of the second heating unit 2; the inner surface of the first heating unit 1 is provided with a second guide port 102 that is inwards sunken; and the insertion block 31 on the second heating unit 2 can pass through the second guide port 102 and be inserted into the insertion slot 320 on the first hand warmer unit.

Specifically, the second guide port 102 and the first guide port 201 can respectively limit and guide the insertion block 31 on the second heating unit 2 and the insertion block 31 on the first heating unit 1, so that the corresponding insertion blocks 31 can be accurately and quickly inserted into the corresponding insertion slots 320. This ensures the assembling accuracy between the first heating unit 1 or the second heating unit 2, improves the convenience of use, and provides a user with a feedback to indicate a current position of the first heating unit 1 or the second heating unit 2, thus improving the operating handfeel.

Referring to FIG. 6 to FIG. 9, a first hidden slot 101 is arranged on the first heating unit 1; when the connection block 32 on the second heating unit 2 is placed in the first hidden slot 101, the insertion block 31 on the first heating unit 1 is inserted into the insertion slot 320 on the second heating unit 2; a second hidden slot 202 is arranged on the second heating unit 2; and when the connection block 32 on the first heating unit 1 is placed in the second hidden slot 202, the insertion block 31 on the second heating unit 2 is inserted into the insertion slot 320 on the first heating unit 1.

Since the first hidden slot 101 can sleeve and limit the connection block 32 on the second heating unit 2, and the second hidden slot 202 can sleeve and limit the connection block 32 on the first heating unit 1, on the one hand, this can further improve the stability of the first heating unit 1 and the second heating unit 2 after assembling; and on the other hand, this can also avoid protruding portions on two edges of the assembled hand warmer, thus ensuring the overall aesthetics and making it convenient for a user to hold and carry the hand warmer.

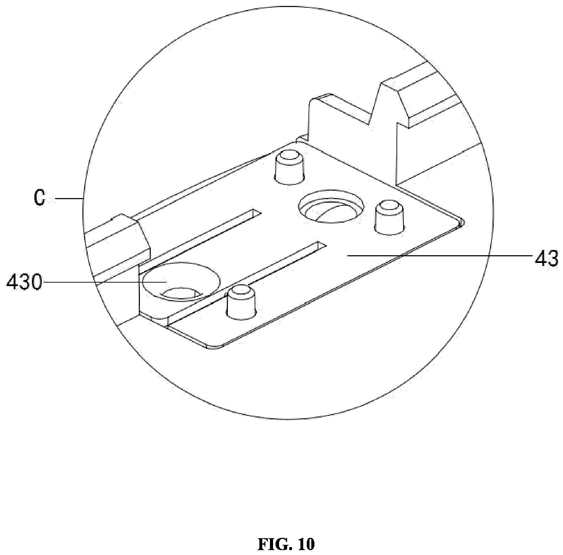

Referring to FIG. 7 to FIG. 12, the combined type hand warmer includes two locking portions 4; the elastic sheets 43 of the two locking portions 4 are respectively mounted inside the first heating unit 1 and the second heating unit 2, and the two elastic sheets 43 are staggered from each other; and the movement holes 41 and lock holes 42 of the two locking portions 4 are distributed on the inner surface of the first heating unit 1 and the inner surface of the second heating unit 2 to respectively adapt to the locking pieces 430 of the two elastic sheets 43.

Specifically, in the process of inserting insertion blocks 31 into the insertion slots 320, the inner surface of the first heating unit 1 pushes the locking piece 430 on the second heating unit 2 into the movement hole 41 on the second heating unit 2, and the inner surface of the second heating unit 2 simultaneously pushes the locking piece 430 on the first heating unit 1 into the movement hole 41 on the first heating unit 1. After the insertion blocks 31 are inserted into the insertion slots 320, the locking piece 430 on the first heating unit 1 protrudes out of the movement hole 41 on the first heating unit 1 and is clamped to the lock hole 42 on the second heating unit 2, and the locking piece 430 on the second heating unit 2 also protrudes out of the movement hole 41 on the second heating unit 2 and is clamped to the lock hole 42 on the first heating unit 1.

In the assembled hand warmer, the relative rotation between the first heating unit 1 and the second heating unit 2 is mainly limited by the cooperation between the insertion blocks 31 and the insertion slots 320. This will increase the load on the insertion blocks 31 and the insertion slots 320 and affect the stability after connection. The two staggered elastic sheets 43 can simultaneously form two locking points on the first heating unit 1 and the second heating unit 2. The two locking points can limit the inner surface of the first heating unit 1 and the inner surface of the second heating unit 2 on the same straight line to prevent rotation between the first heating unit 1 and the second heating unit 2. This further improves the overall structural stability.

Further, the combined type hand warmer includes four locking portions 4 to respectively correspond to top and bottom sides of the first heating unit 1 and top and bottom sides of the second heating unit 2. This design can simultaneously form four locking points on the first heating unit 1 and the second heating unit 2. The four locking points can limit the inner surface of the first heating unit 1 and the inner surface of the second heating unit 2 to the same plane to prevent rotation and offset between the first heating unit 1 and the second heating unit 2. This further improves the overall structural stability.

Referring to FIG. 7 to FIG. 12, the locking pieces 430 are hemispherical. This design makes surfaces of the locking pieces 430 smooth and can effectively avoid the exposed locking pieces 430 from scratching a user.

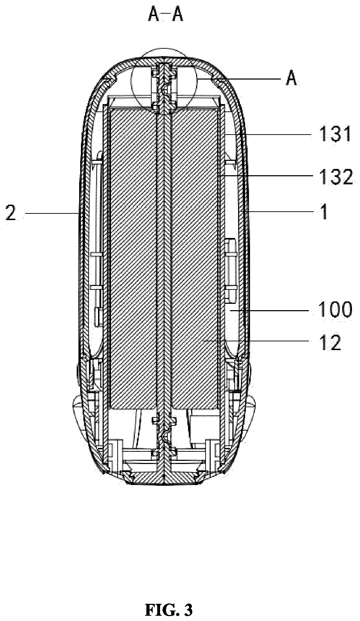

Referring to FIG. 3 and FIG. 9 to FIG. 11, the first heating unit 1 includes a shell 10, a shell cover 11, a battery 12, and a heating element 13; the shell cover 11 is mounted on the shell 10 and is enclosed to form a an accommodating cavity 100; the battery 12 and the heating element 13 are both mounted in the accommodating cavity 100; the battery 12 is electrically connected to the heating element 13; and the second heating unit 2 has the same structure as that of the first heating unit 1.

Specifically, during use, the heating element 13 is powered through the battery 12, so that the heating element 13 can dissipate heat, and a user can hold the shell 10 for getting warm. The cooperation design of the shell 10 and the shell cover 11 facilitates the mounting and removal of the battery 12 and the heating element 13, thus facilitating the maintenance.

Further, the heating element 13 is mainly composed of a circuit board 131, an electric heating plate 132, a control button 133, a charging end 134, a display lamp 135, and other elements. The circuit board 131 and the electric heating plate 132 are both mounted in the accommodating cavity 100, and the control button 133, the charging end 134, and the display lamp 135 are mounted on the shell 10 or the shell cover 11, and the various elements are electrically connected to each other.

A user can charge the battery 12 through the charging end 134, and can control the battery 12 to supply power to the electric heating plate 132 through the control button 133, thereby enabling the electric heating plate 132 to generate heat for warming. The display lamp 135 is configured to display a battery percentage of the battery 12 or for lighting. The related structures are conventional in the technical field, so that specific circuit structures and working principles will not be elaborated in detail here.

The above describes the preferred embodiments of the present disclosure and is not intended to limit the present disclosure. Any modification, equivalent replacement, and improvement made within the spirit and scope of the present disclosure shall fall within the protection scope of the present disclosure.

## Claims

1. A combined type hand warmer, comprising a first heating unit and a second heating unit, and further comprising:
a connection portion, wherein the connection portion comprises an insertion block and a connection block; the insertion block is arranged on an edge of the first heating unit and extends laterally; the connection block is arranged on an edge of the second heating unit and is provided with an insertion slot with a lateral opening; the insertion block is able to be separated from or inserted into the insertion slot; and
a locking portion, wherein the locking portion comprises a movement hole, a lock hole, and an elastic sheet; the movement hole is arranged on an inner surface of the first heating unit; the lock hole is arranged on an inner surface of the second heating unit; the elastic sheet is mounted inside the first heating unit; the elastic sheet is provided with a locking piece; and the locking piece is located at the movement hole and is able to be separated from or clamped into the lock hole.

2. The combined type hand warmer according to claim 1, wherein the insertion block on the first heating unit protrudes out of the inner surface of the first heating unit; the inner surface of the second heating unit is provided with a first guide port that is inwards sunken; and the insertion block on the first heating unit is able to pass through the first guide port and be inserted into the insertion slot on the second heating unit.

3. The combined type hand warmer according to claim 1, wherein a first hidden slot is arranged on the first heating unit; and when the connection block on the second heating unit is placed in the first hidden slot, the insertion block on the first heating unit is inserted into the insertion slot on the second heating unit.

4. The combined type hand warmer according to claim 1, wherein the combined type hand warmer comprises two connection portions; the insertion blocks of the two connection portions are respectively arranged on an edge of the first heating unit and an edge of the second heating unit; the connection blocks of the two connection portions are respectively arranged on the first heating unit and the second heating unit; and when the insertion block on the first heating unit is inserted into the insertion slot on the second heating unit, the insertion block on the second heating unit is synchronously inserted into the insertion slot on the first heating unit.

5. The combined type hand warmer according to claim 4, wherein the insertion block on the first heating unit protrudes out of the inner surface of the first heating unit; the inner surface of the second heating unit is provided with a first guide port that is inwards sunken; the insertion block on the first heating unit is able to pass through the first guide port and be inserted into the insertion slot on the second heating unit;
the insertion block on the second heating unit protrudes out of the inner surface of the second heating unit; the inner surface of the first heating unit is provided with a second guide port that is inwards sunken; and the insertion block on the second heating unit is able to pass through the second guide port and be inserted into the insertion slot on the first heating unit.

6. The combined type hand warmer according to claim 4, wherein a first hidden slot is arranged on the first heating unit; when the connection block on the second heating unit is placed in the first hidden slot, the insertion block on the first heating unit is inserted into the insertion slot on the second heating unit;
a second hidden slot is arranged on the second heating unit; and when the connection block on the first heating unit is placed in the second hidden slot, the insertion block on the second heating unit is inserted into the insertion slot on the first heating unit.

7. The combined type hand warmer according to claim 1, wherein the combined type hand warmer comprises two locking portions; the elastic sheets of the two locking portions are respectively mounted inside the first heating unit and the second heating unit, and the two elastic sheets are staggered from each other; and the movement holes and lock holes of the two locking portions are distributed on the inner surface of the first heating unit and the inner surface of the second heating unit to respectively adapt to the locking pieces of the two elastic sheets.

8. The combined type hand warmer according to claim 7, wherein the combined type hand warmer comprises four locking portions to respectively correspond to top and bottom sides of the first heating unit and top and bottom sides of the second heating unit.

9. The combined type hand warmer according to claim 8, wherein the locking pieces are hemispherical.

10. The combined type hand warmer according to claim 1, wherein the first heating unit comprises a shell, a shell cover, a battery, and a heating element; the shell cover is mounted on the shell and is enclosed to form a an accommodating cavity; the battery and the heating element are both mounted in the accommodating cavity; the battery is electrically connected to the heating element; and the second heating unit has the same structure as that of the first heating unit.

## Amended claims

### Amended claims in accordance with Rule 137(2) EPC.

1. A combined type hand warmer, comprising a first heating unit (1), a second heating unit (2), and a connection portion (3),
the connection portion (3) comprises an insertion block (31) and a connection block (32);
the insertion block (31) is arranged on an edge of the first heating unit (1) and extends laterally; the connection block (32) is arranged on an edge of the second heating unit (2) and is provided with an insertion slot (320) with a lateral opening; the insertion block (31) is able to be separated from or inserted into the insertion slot (320);
the combined type hand warmer is **characterized by**:
the combined type hand warmer further comprises a locking portion (4), wherein the locking portion (4) comprises a movement hole (41), a lock hole (42), and an elastic sheet (43); the movement hole (41) is arranged on an inner surface of the first heating unit (1); the lock hole (42) is arranged on an inner surface of the second heating unit (2); the elastic sheet (43) is mounted inside the first heating unit (1); the elastic sheet (43) is provided with a locking piece (430) configured to partially protrude out the first heating unit through the movement hole and to retract into the first heating unit through the movement hole; and the locking piece (430) is moveably arranged in the movement hole (41) and is able to be separated from or clamped into the lock hole (42).

2. The combined type hand warmer according to claim 1, wherein the insertion block (31) on the first heating unit (1) protrudes out of the inner surface of the first heating unit (1); the inner surface of the second heating unit (2) is provided with a first guide port (201) that is inwards sunken; and the insertion block (31) on the first heating unit (1) is able to pass through the first guide port (201) and be inserted into the insertion slot (320) on the second heating unit (2).

3. The combined type hand warmer according to claim 1, wherein a first hidden slot (101) is arranged on the first heating unit (1); and when the connection block (32) on the second heating unit (2) is placed in the first hidden slot (101), the insertion block (31) on the first heating unit (1) is inserted into the insertion slot (320) on the second heating unit (2).

4. The combined type hand warmer according to claim 1, wherein the combined type hand warmer comprises two connection portions (3); the insertion blocks (31) of the two connection portions (3) are respectively arranged on an edge of the first heating unit (1) and an edge of the second heating unit (2); the connection blocks (32) of the two connection portions (3) are respectively arranged on the first heating unit (1) and the second heating unit (2); and when the insertion block (31) on the first heating unit (1) is inserted into the insertion slot (320) on the second heating unit (2), the insertion block (31) on the second heating unit (2) is synchronously inserted into the insertion slot (320) on the first heating unit (1).

5. The combined type hand warmer according to claim 4, wherein the insertion block (31) on the first heating unit (1) protrudes out of the inner surface of the first heating unit (1); the inner surface of the second heating unit (2) is provided with a first guide port (201) that is inwards sunken; the insertion block (31) on the first heating unit (1) is able to pass through the first guide port (201) and be inserted into the insertion slot (320) on the second heating unit (2);
the insertion block (31) on the second heating unit (2) protrudes out of the inner surface of the second heating unit (2); the inner surface of the first heating unit (1) is provided with a second guide port (102) that is inwards sunken; and the insertion block (31) on the second heating unit (2) is able to pass through the second guide port (102) and be inserted into the insertion slot (320) on the first heating unit (1).

6. The combined type hand warmer according to claim 4, wherein a first hidden slot (101) is arranged on the first heating unit (1); when the connection block (32) on the second heating unit (2) is placed in the first hidden slot (101), the insertion block (31) on the first heating unit (1) is inserted into the insertion slot (320) on the second heating unit (2);
a second hidden slot (202) is arranged on the second heating unit (2); and when the connection block (32) on the first heating unit (1) is placed in the second hidden slot (202), the insertion block (31) on the second heating unit (2) is inserted into the insertion slot (320) on the first heating unit (1).

7. The combined type hand warmer according to claim 1, wherein the combined type hand warmer comprises two locking portions (4); the elastic sheets (43) of the two locking portions (4) are respectively mounted inside the first heating unit (1) and the second heating unit (2), and the two elastic sheets (43) are staggered from each other; and the movement holes (41) and lock holes (42) of the two locking portions (4) are distributed on the inner surface of the first heating unit (1) and the inner surface of the second heating unit (2) to respectively adapt to the locking pieces (430) of the two elastic sheets (43).

8. The combined type hand warmer according to claim 7, wherein the combined type hand warmer comprises four locking portions (4) to respectively correspond to top and bottom sides of the first heating unit (1) and top and bottom sides of the second heating unit (2).

9. The combined type hand warmer according to claim 8, wherein the locking pieces (430) are hemispherical.

10. The combined type hand warmer according to claim 1, wherein the first heating unit (1) comprises a shell (10), a shell cover (11), a battery (12), and a heating element (13); the shell cover (11) is mounted on the shell (10) and is enclosed to form a an accommodating cavity (100); the battery (12) and the heating element (13) are both mounted in the accommodating cavity (100); the battery (12) is electrically connected to the heating element (13); and the second heating unit (2) has the same structure as that of the first heating unit (1).
